# FASCICULE DE BREVET EUROPEEN

(11) **EP 1 698 304 B1**
(45) Date de publication et mention de la délivrance du brevet: **23.04.2008**
(21) Numéro de dépôt: 06290324.0
(22) Date de dépôt: 28.02.2006
(51) Int. Cl.: A61F 2/36

(54) **Prothèse à aspérités auto-impactable**
Prothese mit selbststanzenden Erhöhungen
Prosthesis with self-impacting asperities

(30) Priorité: 01.03.2005 FR 0502076
(43) Date de publication de la demande: 06.09.2006
(73) Titulaire: A.T.S. SFERIC S.A.S., 41500 Menars (FR)
(72) Inventeur: Costa, José, 92130 Issy-les-Moulineaux (FR); Cousin, Thierry, 41500 Menars (FR); Grimard, Jean-Christophe, 41120 Cellettes (FR)
(74) Mandataire: Debay, Yves

(56) Documents cités:
- DE-A1- 2 839 092
- FR-A- 2 800 599
- US-A- 3 894 297
- US-A- 4 608 053
- US-A- 4 863 474
- US-A- 4 944 763

## Description

La présente invention concerne une endoprothèse destinée à être placée dans un canal intra-médullaire.

En arthroplastie articulaire et quelle que soit l'articulation, il existe plusieurs types de prothèses qui diffèrent :
- selon la pathologie à traiter (traumatisme, arthrose, malformation,...),
- selon l'état de santé du patient,
- selon le type de voie d'abord et de technique opératoire (voie antérieure, voie postérieure, avec ou sans trochantérotomie...),
pouvant engendrer des modifications au niveau :
- du type de géométrie de la prothèse (forme autobloquante ou non, anatomique ou droite, avec ou sans platine d'appui, section ovoïde ou rectangulaire,...),
- du type de fixation (avec ou sans ciment, avec ou sans revêtement, ...)

Les chirurgiens souhaitent adapter la préparation du canal intra-médullaire de l'os, en fonction du type de prothèse qu'ils souhaitent ensuite disposer, afin d'assurer un geste opératoire efficace et sans risque, tout en garantissant la fiabilité de la fixation de l'implant.

Aussi pour une bonne réussite de l'implant, il faut d'abord que le calibrage du canal intra-médullaire soit très précis, il faut ensuite un excellent accrochage osseux de l'implant. Ces deux caractéristiques sont primordiales pour assurer le maintien à long terme de la prothèse en particulier si elle est fixée sans ciment.

Le calibrage de l'os est généralement effectué à l'aide d'une râpe. Celle-ci façonne le canal intra-médullaire avec précision pour que le volume obtenu soit en correspondance avec le volume de l'implant à mettre en place.

II est connu différents produits destinés à calibrer le canal intra-médullaire :

Par exemple, la demande de brevet EP 1 174 201 A1 expose une râpe constituée d'une paroi extérieure sur laquelle des aspérités ont été usinées pour râper la paroi interne du canal.

Il est connu également pour le brevet US 5, 665, 091 une râpe pour préparer le canal intra-médullaire d'un os destiné à recevoir une prothèse.

Par ailleurs, jusqu'à présent toutes les prothèses sont lisses pour éviter leurs fragilisations causées par les amorces de ruptures et de leur donner ainsi une bonne tenue dans le temps. En effet, il n'est pas rare que des prothèses cassent en service du fait de leurs fragilisations causées par ces amorces de ruptures.

L'inconvénient de procéder avec les produits de l'art antérieur, est qu'il existe une probabilité d'erreur de jeu entre le calibre du logement creusé par le chirurgien avec l'aide de la râpe et le calibre de la prothèse destiné à s'insérer dans ledit logement.

Par ailleurs, en procédant avec les produits de l'art antérieur, le maintien de la prothèse par l'os à l'intérieur du canal intra-médullaire est souvent mauvais du fait d'un faible accrochage osseux des prothèses. A ce jour la déstabilisation de l'implant fémoral est une des principales causes d'échec de l'arthroplastie de la hanche.

Des aspérités sont parfois prévues pour améliorer l'insertion et le maintien des implants. Les documents US 4 944 763, US 4 608 053, US 3 894 297, FR 2 800 599, DE 28 39 092 et US 4 863 474 divulguent ainsi des systèmes doté d'aspérités.

La présente invention a donc pour objet de pallier les inconvénients de l'art antérieur en proposant une prothèse capable d'auto-calibrer parfaitement son logement tout en présentant un excellent accrochage osseux.

Ce but est atteint par une prothèse intra-médullaire selon l'enseignement de la revendication 1.

Selon une autre particularité, les aspérités sont réalisées par un outil biseauté dont le plan de symétrie est déplacé perpendiculairement aux génératrices du solide de matériau selon une hélice.

Selon une autre particularité, les aspérités sont réalisées par un outil biseauté dont le plan de symétrie est déplacé perpendiculairement aux génératrices du solide de matériau selon deux hélices à pas inversé se croisant.

Selon une autre particularité, les aspérités sont réalisées par un outil biseauté dont le plan de symétrie est déplacé perpendiculairement aux génératrices du solide de matériau selon deux hélices de même sens et de pas ou d'inclinaison différente.

Selon d'autres particularités, la prothèse est telle que définie dans les revendications 2 à 10.

L'invention, avec ses caractéristiques et avantages, ressortira plus clairement à la lecture de la description faite en référence aux dessins annexés dans lesquels :
- la figure 1 représente, en vue de côté de la prothèse selon l'invention sur laquelle sont tracées quelques génératrices,
- la figure 2 représente une portion la prothèse en perspective sur laquelle est tracée une portion de courbe en hélice servant à l'usinage des aspérités de la prothèse,
- la figure 3 représente, en vue de côté et simplifiée, la prothèse avant usinage, sur laquelle a été tracée une courbe en forme d'hélice,
- la figure 4 représente, en vue de côté, l'outil utilisé dans l'usinage des aspérités,
- la figure 5 représente, en perspective, le corps d'une prothèse usiné partiellement suivant une hélice de pas constant,
- la figure 6 représente, en vue de côté et simplifiée, la prothèse à la suite d'un usinage,
- la figure 7 représente, en vue de côté et simplifiée, la prothèse sur laquelle a été tracée deux courbes en hélice croisées,
- la figure 8 représente, en vue de côté et simplifiée, la prothèse après un exemple d'usinage suivant deux courbes en hélice croisées et la représentation simplifiée des aspérités.
- la figure 9 représente une section de la prothèse suivant l'inclinaison d'une courbe en hélice après un usinage suivant deux courbes en hélice croisées,
- la figure 10 représente, en vue de côté, les dents obtenues après l'exemple d'usinage suivant les deux hélices croisées,
- la figure 11 représente, en perspective, une dent en forme de pyramide régulière.

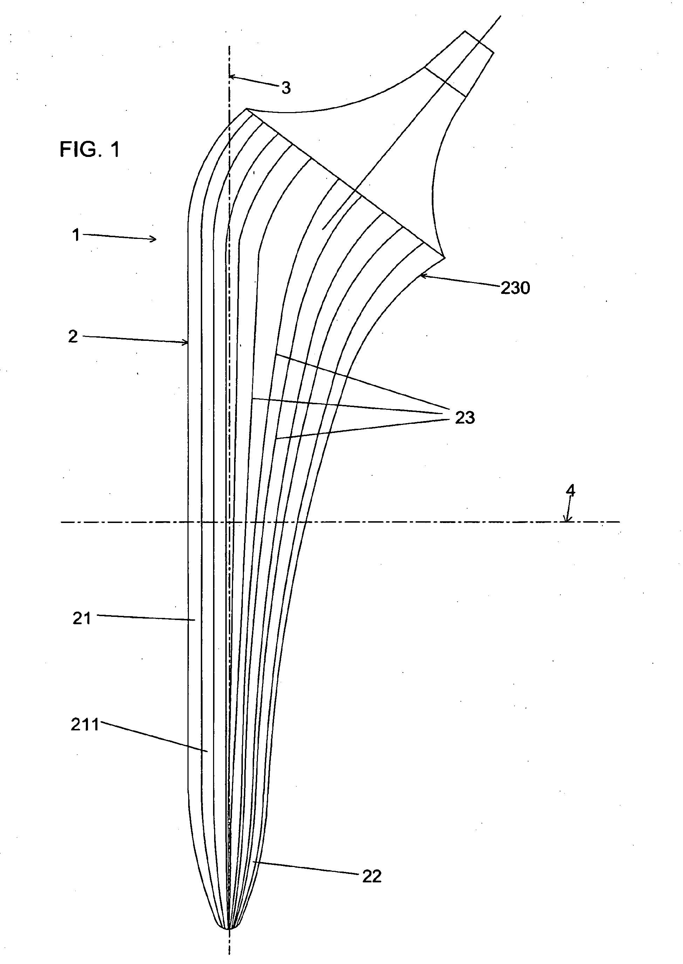

La prothèse selon l'invention sera d'abord décrite en liaison avec les figures 1 à 6.

Une prothèse (1), selon l'invention, est destinée à être insérée à l'intérieur du canal intra-médullaire d'un os. La prothèse (1) ici présentée est appropriée pour s'auto-calibrer dans le canal du fémur grâce aux aspérités saillantes qui permettent à la prothèse de râper le canal intra-médullaire. Chaque prothèse (1) a une forme variable selon la forme du canal intra-médullaire de l'os que l'utilisateur souhaite soigner.

Selon une forme connue, une prothèse est constituée d'un corps (2) comprenant une partie supérieure (20) et une paroi extérieure (21) lisse. Le corps (2) se termine par une forme profilée (22) en fuseau destinée à faciliter l'enfoncement de la prothèse (1) dans le canal.

Selon notre invention, le corps (2) de la prothèse (1) a une forme allongée selon un axe (3) sensiblement parallèle à l'axe longitudinal du canal. La section transversale du corps (2) de la prothèse (1) est variable le long du corps (2), puis décroissante sur son extrémité finale, pour se terminer par la forme profilée (22) destinée à faciliter l'enfoncement de la prothèse (1). La forme de la section transversale de la partie inférieure (211) de la prothèse (1) avant la forme en fuseau (22) est sensiblement constante. Sur la partie supérieure du corps (2) de la prothèse (1), la forme de la section transversale du corps (2) est progressivement variable et supérieure à la section transversale de la partie inférieure (211) du corps (2). La partie supérieure (20) du corps (2) peut être utilisée comme moyen de préhension pour manipuler la prothèse (1). Le corps (2) comporte enfin des aspérités suffisamment saillantes, au moins dans la partie en fuseau (22) et le début de la partie inférieure (211) adjacentes, pour pouvoir râper le canal intra-médullaire de l'os et pour pouvoir assurer un bon accrochage osseux, par auto impactage de la prothèse dans l'os.

Ainsi le calibrage du canal intra-médullaire pour la prothèse est très précis car c'est l'implant qui creuse son propre logement. L'outil traditionnel de calibrage n'est plus nécessaire afin dé calibrer le canal intra-médullaire, seul des outils de préparation de base restent nécessaires.

Le maintien de la prothèse est assuré par la fixation primaire et la fixation secondaire. D'abord, comme c'est la prothèse qui calibre son logement, la fixation primaire est assurée par l'élasticité de l'os intra-médullaire qui fait pression sur la prothèse dans son logement et maintient ainsi la prothèse en place. Ensuite la fixation secondaire est assurée par l'accrochage osseux assuré par la réhabitation de l'os dans les interstices des aspérités de la prothèse. Cette fixation secondaire assure le maintien à long terme de la prothèse.

Par ailleurs, en fonction des différents types d'os du canal intra-médullaire, les aspérités doivent être plus ou moins tranchantes ou plus ou moins tassantes pour s'adapter aux différents types d'os. Certaines aspérités doivent présenter à la fois un bon accrochage osseux et permettre une extraction relativement aisée de l'implant s'il y a une réintervention chirurgicale.

Pour réaliser ces aspérités au caractère tranchant ou tassant souhaité, il existe différents procédés d'usinage. Cependant l'homme du métier s'est souvent refusé à usiner des aspérités sur une prothèse par crainte de former une amorce de rupture sur la prothèse et de favoriser la présence de zone de concentration des contraintes en service. Des nouveaux procédés d'usinage permettent d'usiner ces aspérités sans fragiliser la prothèse.

Selon un premier exemple, on peut réaliser les aspérités en usinant suivant une hélice (210)

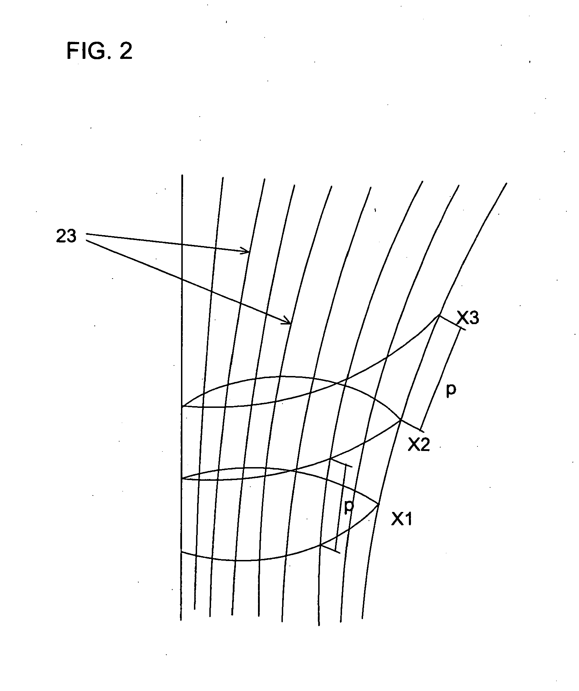

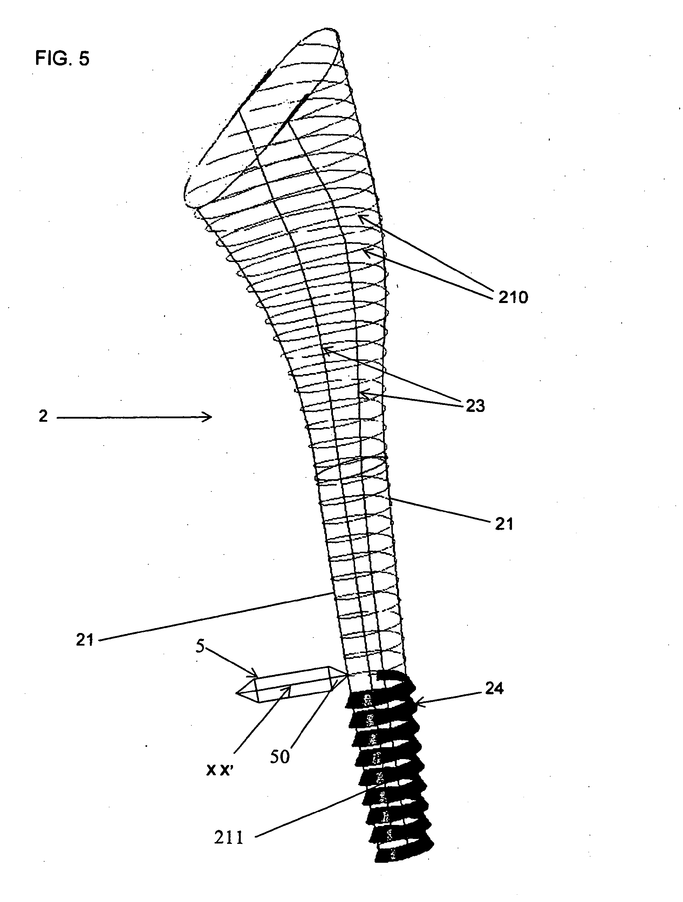

Dans l'exemple d'usinage une première courbe en hélice (210) est une représentation de la paroi extérieure (21) du corps (2) de la prothèse (1). La courbe en hélice (210) est obtenue en positionnant, des points X sur la génératrice (23) distants entre eux du pas constant p comme représenté sur la figure 2 et 5.

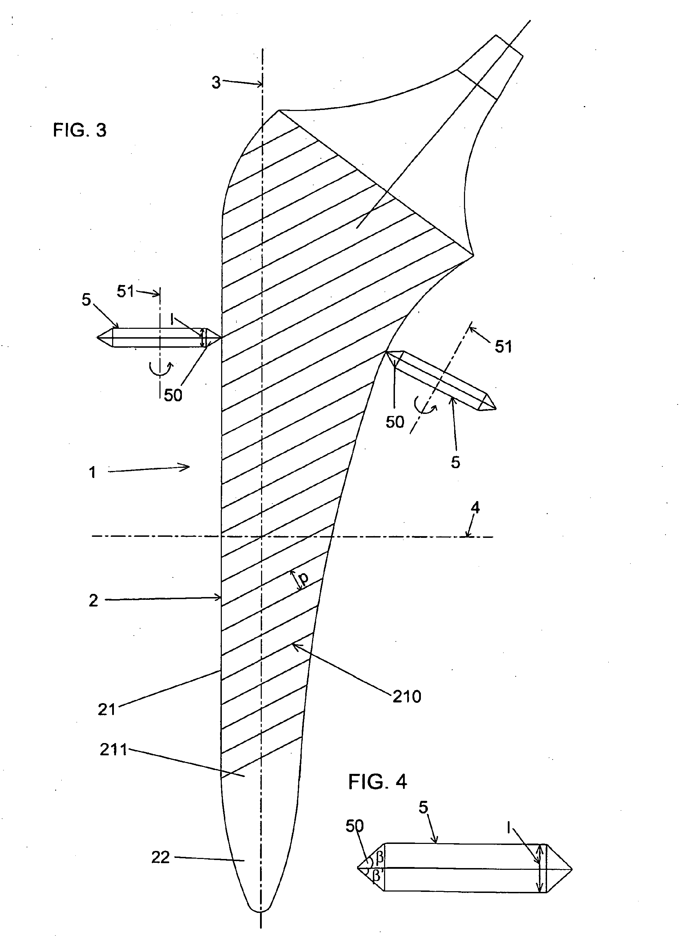

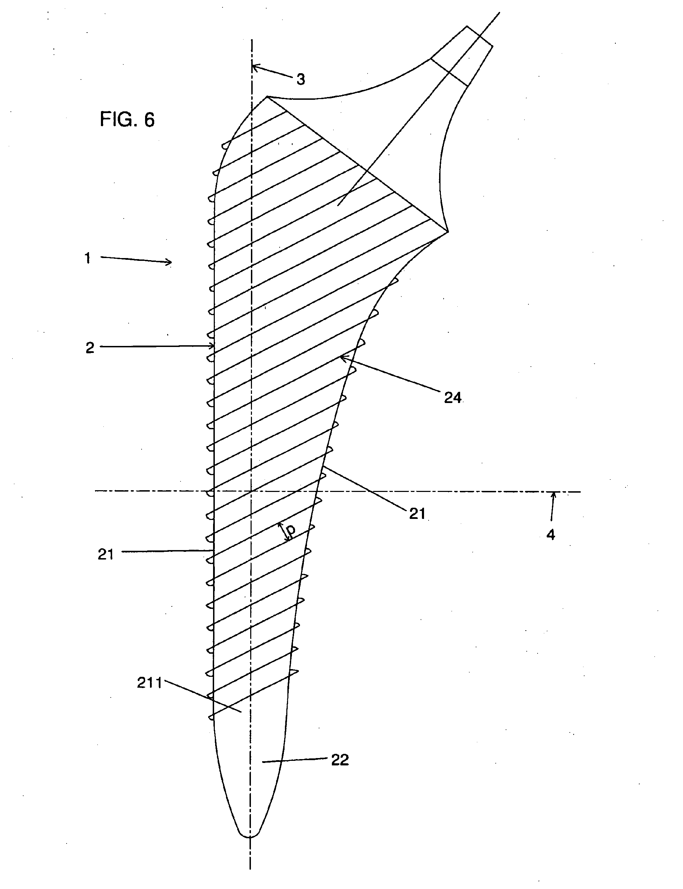

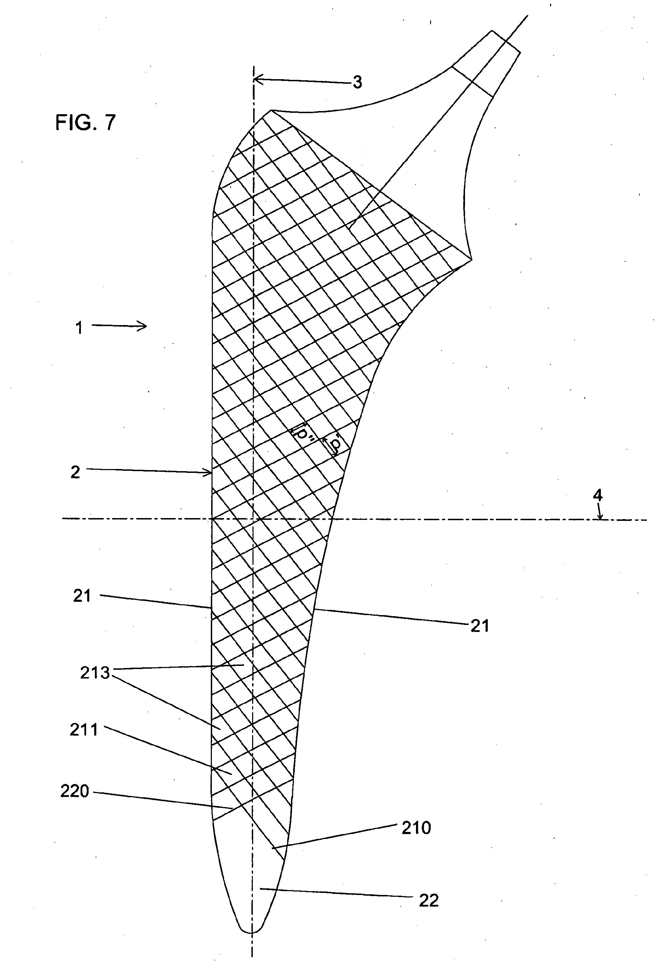

La figure 3 est une représentation simplifiée de la prothèse sur laquelle est tracée une courbe en hélice de pas constant. En effet, le pas de la courbe en hélice que l'on souhaite représenter étant constant et la section transversale du corps (2) de la prothèse (1) étant variable, l'angle de la courbe hélice, défini par rapport à l'axe (4) perpendiculaire à l'axe (3) de la prothèse (1), n'est en réalité pas constant comme cela est représenté sur cette figure. La section transversale dans la partie supérieure du corps (2) de la prothèse (1) étant supérieure à celle de la partie inférieure (211) du corps (2) de la prothèse (1), l'angle de la courbe en hélice varie en décroissant du bas vers le haut du corps (2) de la prothèse (1). Les représentations sont également simplifiées sur les figures 6, 7 et 8.

Dans l'exemple du cas d'un pas constant, l'angle de la courbe en hélice est variable le long du corps (2) de la prothèse (1), mais on parlera tout de même d'inclinaison d'hélice correspondant à l'angle maximum de la courbe en hélice. Cet angle est maximum là où la section transversale du corps (2) de la prothèse (1) est la plus petite, c'est à dire sur la partie inférieure (211) du corps (2) de la prothèse (1).

Une fois l'hélice (210) dessinée les valeurs numériques des trajets définies sont communiquées à la machine d'usinage pour débuter suivant le tracé de la courbe en hélice, comme représenté figure 5. L'usinage est réalisé avec un outil (5) représenté figure 5, fraise ou meule, ayant une tête (50) d'attaque de forme biseautée. Cette tête (50) peut avoir des largeurs 1 différentes et un biseau dont l'angle, défini par β et β' égaux ou différents, peut varier suivant le caractère plus ou moins tranchant que l'on souhaite donner aux aspérités de la prothèse (1). La profondeur d'usinage varie en fonction de la valeur de la largeur I et de l'angle de la tête biseautée. La fraise ou meule, est entraînée en rotation selon un axe (51) maintenu en permanence parallèle aux tangentes aux points appartenant aux génératrices (23) successives et formant la courbe en hélice (210), comme représenté figures 3 et 5 ou en maintenant le plan XX' perpendiculaire à la génératrice.

Une fois l'usinage suivant l'hélice (210) terminé sur la hauteur de prothèse souhaitée, le corps (2) de la prothèse (1) présente un filet (24), comme représenté figures 5 et 6.

Ce filet (24) (figure 5) en spirale continue possède le surprenant avantage de limiter les risques de rupture, car les efforts de travail ne sont plus concentrés uniquement sur la section la plus faible mais ils se répartissent sur toute la hauteur de la denture et ce d'autant que l'angle de l'hélice de la spirale est grand. Ainsi les risques que la prothèse casse en service sont limités. De plus on peut limiter la portion de la prothèse jouant le rôle de râpe à la zone représentée en gras et correspondant à la partie fuselée et le début de la partie inférieure (211) adjacente. La profondeur du filet diminuant progressivement. Ainsi les zones de plus faibles sections sont de moins en moins entaillées par l'usinage des aspérités ; les contraintes qui y sont exercé sont plus faibles.

Ainsi, dans cette exemple d'usinage, si l'usinage est réalisé suivant une hélice (210) d'inclinaison faible, la prothèse dispose alors d'une denture du type denture droite. Cette denture a le caractère d'être tranchant dans l'os du canal intra-médullaire.

Au contraire si l'on souhaite des aspérités tassantes on choisira un angle de biseau pour l'outil très aigu pour obtenir des aspérités tronquées à face supérieure plane.

Aussi, dans cet exemple d'usinage la largeur de la tête (50) de l'outil (5) en forme de biseau, définie par I, pourra, par exemple, être sensiblement égale ou supérieure au pas p de l'hélice (210), de manière à former un filet (24) coupant et donc plus tranchant. Ainsi, on peut adapter la prothèse à différent type d'os en adaptant le caractère tranchant.

Dans un autre exemple d'usinage il est possible d'usiner la prothèse suivant plusieurs hélices de même sens mais d'inclinaisons différentes et décalées le long de l'axe (3) de la prothèse (1). Dans ce cas, l'usinage peut être réalisé à une profondeur différente suivant chaque hélice pour former ainsi une denture dite avec brise-copeaux. Ce qui rend la denture plus tranchante dans l'os du canal intra-médullaire.

Dans un autre exemple, on peut aussi usiner suivant deux courbes en hélice (210, 220) de pas inversé sur la paroi extérieure (21) du corps (2) de la prothèse (1) pour obtenir des aspérités en forme de denture ayant une base pyramidale (215) comme présenté sur les figures de 7 à 11. Ce qui rend la denture plus tranchante dans l'os du canal intra-médullaire.

On peut ainsi réaliser un filet coupant pour former une denture pyramidale en choisissant la tête (50) de l'outil (5) de manière à ce que sa largeur I soit sensiblement égale ou supérieure au pas (p', p") de l'hélice (210, 220) qu'il suit. La denture en forme de pyramides (215) ayant un sommet en pointes confère un effet tranchant aux aspérités dans l'os.

Selon l'exemple ci-dessus on peut obtenir dans une autre variante une denture en brise-copeaux. Pour cela, il faut que les deux profondeurs d'usinage suivant respectivement les deux hélices (210, 220) soient différentes.

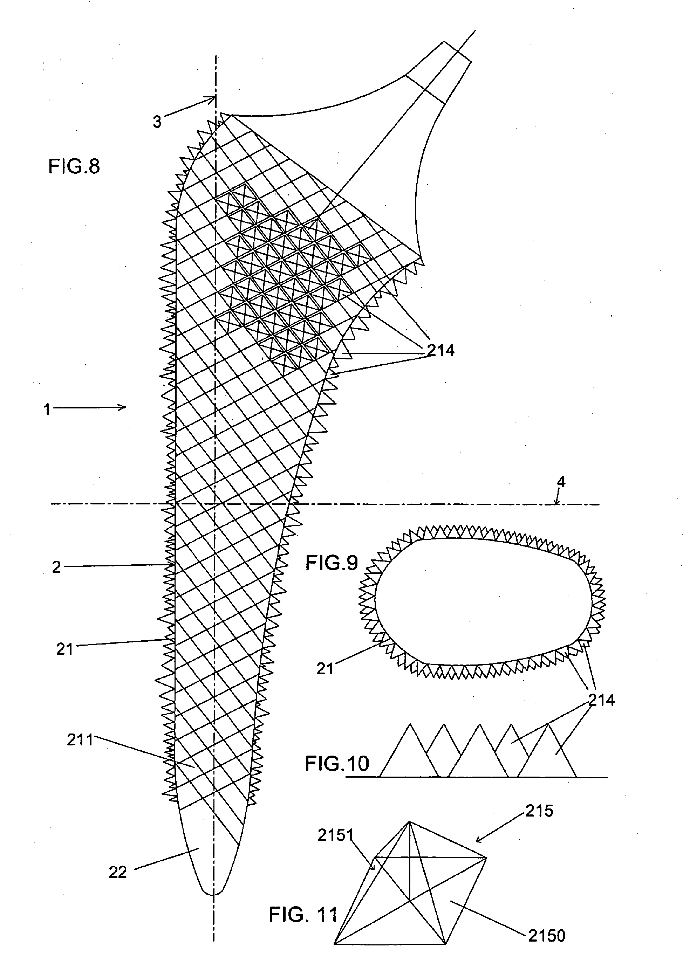

On peut ainsi réaliser une denture en pyramides régulières (215), comme représenté figures 10 et 11 en usinant suivant les hélices (210, 220) de pas inversés à l'aide d'une tête (50) d'un outil (5) en forme en biseau, ayant β et β' égaux, de pas p' et p" égaux et ayant une profondeur de pénétration de l'outil (5) constante et égale suivant les deux hélices (210, 220). Ce qui rend la denture plus tranchante dans le canal intra-médullaire.

Ces pyramides (215) ont pour bases (2150) les espaces (213) formés par les intersections des deux hélices (210, 220) de pas inversés et leur hauteur est normale à la surface du corps (2) de la prothèse (1). Ces dents (214) en forme de pyramides régulières (215) sont en formes de pointes de diamant et sont très tranchantes.

Aussi, l'angle de la tête (50) en biseau défini par β et β' pourra, par exemple, être de 90° de manière à former des pyramides (215) dont les faces latérales (2151) font un angle de 45° avec la base.

Il est possible également de réaliser des pyramides tronquées ou pourvues d'une calotte sur leurs parties supérieures en incluant dans le profil de la tête (50) biseautée de l'outil (5) une partie plane ou rayonnée. Cela rendra la denture moins agressive et produira un effet plus tassant dans l'os.

Aussi si les inclinaisons des hélices choisies sont différentes, les dents (214) pyramidales formées seront toutes décalées suivant l'axe (3) de la prothèse (1). Le calibrage complet de toute la paroi intra-médullaire est alors réalisé, en évitant la formation de stries verticales dans l'os.

Il est aussi possible de former des dents de type pyramidales irrégulières sur certaines parties de la surface de la prothèse en inclinant la tête (50) de l'outil (5) par rapport à sa position normale à la surface. Ces irrégularités auront pour effet de changer localement le caractère tranchant des aspérités de la prothèse.

Il est aussi possible de former des dents de type pyramidales décalées en jouant sur la valeur des angles β et β' de la tête en biseau, c'est à dire avec β différent de β'. Cela permet d'orienter les dents dans une direction prévue en opposition au sens de la coupe pour augmenter le caractère tranchant des dents.

Sur la figure 9, la hauteur des dents est représentée irrégulière car les dents (214) les plus hautes sont celles du premier plan et les plus basses celles de l'arrière plan. Il faut bien comprendre que, par rapport à un contour de prothèse (1) correspondant à une section suivant l'inclinaison d'une hélice (210, 220) comme représenté à la figure 9, la hauteur des dents (214) est constante sur ce contour dans le cas où les deux pas p' et p" sont constants et la pénétration de l'outil (5) également.

Comme exemple de paramétrage de denture pour obtenir des aspérités tranchantes on peut paramétrer une longueur L de 150 mm, une section D de 12 mm, un pas p' de 3 mm, un pas p" de 3 mm également, des angles α' et α" respectivement de 30° et de 50° et un rayon de fond de dent de 0,5 mm.

Une prothèse selon l'invention a en outre la caractéristique d'être réalisée en biomatériau implantable dans le canal intra-médullaire de l'os comme par exemple un acier inoxydable (NF ISO 5832-9) ou un Titane TAGV (NF ISO 5832-4) ou un chrome cobalt (NF ISO 5832-12).

Il est bien évident que différentes combinaisons des modes de réalisations sont possibles.

Aussi on peut envisager de réaliser la variante à double hélices à pas inversé sur une position inférieure de la prothèse, les extrémités des hélices pénétrant de moins en moins dans le matériau aux extrémités de l'hélice. Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Prothèse intra-médullaire comprenant un corps allongé parallèle à l'axe longitudinal (3) du canal intra-médullaire de l'os à opérer, le corps allongé étant à section transversale variable, une paroi extérieure (21) apte à collaborer avec la paroi interne du canal intra-médullaire de l'os, la paroi extérieure (21) étant en biomatériau implantable dans le canal intra-médullaire, des aspérités réparties sur la paroi extérieure (21) au moins sur une zone en contact avec la paroi interne du canal intra-médullaire de l'os, **caractérisée en ce que** les aspérités forment une denture droite en forme de filet (24) tranchant et continu selon deux spirales, et sont suffisamment saillantes pour permettre de râper la paroi interne du canal intra-médullaire de l'os tout en limitant la fragilisation de la prothèse.

2. Prothèse intra-médullaire selon la revendication 1 **caractérisée en ce que** les aspérités forment une denture droite conférant aux aspérités un effet tranchant dans l'os.

3. Prothèse intra-médullaire selon la revendication 1 **caractérisée en ce que** les deux spirales (210, 220) sont à pas inversé et se croisent, pour augmenter la répartition des contraintes exercées sur la prothèse (1) et diminuer la concentration des contraintes.

4. Prothèse intra-médullaire selon la revendication 1 **caractérisée en ce que** les deux spirales sont de même sens et de pas ou d'inclinaison différente, pour augmenter la répartition des contraintes exercées sur la prothèse (1) et diminuer la concentration des contraintes.

5. Prothèse intra-médullaire selon la revendication 1 **caractérisée en ce que** les aspérités forment une denture dont les dents ont la base d'une pyramide (215), ceci conférant aux aspérités un effet tranchant dans l'os.

6. Prothèse intra-médullaire selon la revendication 1 **caractérisée en ce que** les aspérités forment une denture en forme de pyramides (215) ayant un sommet en pointes conférant aux aspérités un effet tranchant dans l'os.

7. Prothèse intra-médullaire selon une des revendications 5 et 6 **caractérisée en ce que** les pyramides (215) sont aplanies sur leurs sommets.

8. Prothèse intra-médullaire selon une des revendications 5 à 7, **caractérisée en ce que** les pointes des pyramides (215) sont décalées par rapport au centre de leurs bases (2150) pour que les sommets des pyramides (215) soient orientés dans un sens prévu en opposition au sens de la coupe conférant ainsi un effet encore plus tranchant dans l'os aux aspérités.

9. Prothèse intra-médullaire selon une des revendications 5 à 8, **caractérisée en ce que** les dents (214) pyramidales (215) adjacentes formées sont décalées selon l'axe de la prothèse (1) pour éviter la formation de stries verticales dans l'os.

10. Prothèse intra-médullaire selon une des revendications 5 et 6, **caractérisée en ce que** les dentures forment une denture brise copeaux conférant aux aspérités un effet tranchant dans l'os.

## Claims

1. Intramedullary prosthesis comprising an elongate body parallel to the longitudinal axis (3) of the intramedullary canal of the bone to be operated upon, the elongate body having a variable cross-section, an outer wall (21) capable of collaborating with the inner wall of the intramedullary canal of the bone, the outer wall (21) being made from biomaterial which can be implanted in the intramedullary canal, irregularities distributed over the outer wall (21) at least over a zone in contact with the inner wall of the intramedullary canal of the bone, **characterised in that** the irregularities form a straight toothing in the form of a continuous cutting thread (24) in two spirals, and project sufficiently to enable rasping of the inner wall of the intramedullary canal of the bone whilst limiting the embrittlement of the prosthesis.

2. Intramedullary prosthesis as claimed in Claim 1, **characterised in that** the irregularities form a straight toothing which gives the irregularities a cutting effect in the bone.

3. Intramedullary prosthesis as claimed in Claim 1, **characterised in that** the two spirals (210, 220) have a reverse pitch and intersect in order to increase the distribution of the stresses exerted on the prosthesis (1) and to reduce the concentration of the stresses.

4. Intramedullary prosthesis as claimed in Claim 1, **characterised in that** the two spirals are in the same direction and have different pitch or inclination, in order to increase the distribution of the stresses exerted on the prosthesis (1) and to reduce the concentration of the stresses.

5. Intramedullary prosthesis as claimed in Claim 1, **characterised in that** the irregularities form a toothing of which the teeth are based on a pyramid (215) which gives the irregularities a cutting effect in the bone.

6. Intramedullary prosthesis as claimed in Claim 1, **characterised in that** the irregularities form toothing in the form of pyramids (215) having a pointed apex which gives the irregularities a cutting effect in the bone.

7. Intramedullary prosthesis as claimed in any one of Claims 5 and 6, **characterised in that** the pyramids (215) are flattened on their apexes.

8. Intramedullary prosthesis as claimed in any one of Claims 5 to 7, **characterised in that** the points of the pyramids (215) are offset with respect to the centre of their bases (2150) in order that the apexes of the pyramids (215) are oriented in a direction opposed to the direction of cutting thus giving the irregularities an even more cutting effect in the bone.

9. Intramedullary prosthesis as claimed in any one of Claims 5 to 8, **characterised in that** the adjacent pyramidal (215) teeth (214) formed are offset according to the axis of the prosthesis (1) in order to avoid the formation of vertical striations in the bone.

10. Intramedullary prosthesis as claimed in any one of Claims 5 and 6, **characterised in that** the toothings form a chip breaker toothing which gives the irregularities a cutting effect in the bone.

## Patentansprüche

1. Intramedulläre Prothese, aufweisend: einen langgestreckten Körper parallel zu der Längsachse (3) des intramedullären Kanals des zu operierenden Knochens, wobei der langgestreckte Körper einen variablen Querschnitt aufweist, eine Außenwand (21), die zum Zusammenwirken mit der Innenwand des intramedullären Kanals des Knochens geeignet ist, wobei die Außenwand (21) aus einem biokompatiblen, in den intramedullären Kanal implantierbaren Material ist, Unebenheiten, die auf der Außenwand (21) zumindest in einem Kontaktbereich mit der Innenwand des intramedullären Kanals des Knochens verteilt sind, **dadurch gekennzeichnet, dass** die Unebenheiten eine Außenverzahnung in Form eines scharfen und durchgehenden Gewindes (24) gemäß zweier Schraubenlinien bilden und ausreichend scharf sind, um ein Raspeln der Innenwand des intramedullären Kanals des Knochens zu erlauben, wobei gleichzeitig die Versprödung der Prothese eingeschränkt wird.

2. Intramedulläre Prothese gemäß Anspruch 1, **dadurch gekennzeichnet, dass** die Unebenheiten eine Außenverzahnung bilden, die den Unebenheiten eine Schneidewirkung im Knochen verleiht.

3. Intramedulläre Prothese gemäß Anspruch 1, **dadurch gekennzeichnet, dass** die beiden Schraubenlinien (210, 220) eine entgegengesetzte Ganghöhe aufweisen und sich kreuzen, so dass die Verteilung der auf die Prothese (1) ausgeübten Spannungen verstärkt und die Spannungskonzentration verringert wird.

4. Intramedulläre Prothese gemäß Anspruch 1, **dadurch gekennzeichnet, dass** die beiden Schraubenlinien gleichsinnig sind und eine unterschiedliche Ganghöhe oder Neigung aufweisen, so dass die Verteilung der auf die Prothese (1) wirkenden Spannungen größer und die Spannungskonzentration verringert wird.

5. Intramedulläre Prothese gemäß Anspruch 1, **dadurch gekennzeichnet, dass** die Unebenheiten eine Verzahnung bilden, deren Zähne die Basis einer Pyramide (215) aufweisen, was den Unebenheiten eine Schneidewirkung im Knochen verleiht.

6. Intramedulläre Prothese gemäß Anspruch 1, **dadurch gekennzeichnet, dass** die Unebenheiten eine Verzahnung in Form von Pyramiden (215) mit einer spitzen Spitze bilden, die den Unebenheiten eine Schneidewirkung im Knochen verleiht.

7. Intramedulläre Prothese gemäß einem der Ansprüche 5 und 6, **dadurch gekennzeichnet, dass** die Pyramiden (215) an ihren Spitzen abgeflacht sind.

8. Intramedulläre Prothese gemäß einem der Ansprüche 5 bis 7, **dadurch gekennzeichnet, dass** die Spitzen der Pyramiden (215) relativ zu der Mitte ihrer Basis (2150) versetzt sind, so dass die Spitzen der Pyramiden (215) in einer Richtung entgegengesetzt zu der Richtung des Schnittes ausgerichtet sind, so dass den Unebenheiten eine noch stärkere Schneidewirkung im Knochen verliehen wird.

9. Intramedulläre Prothese gemäß einem der Ansprüche 5 bis 8, **dadurch gekennzeichnet, dass** die gebildeten benachbarten Zähne (214) in Pyramidenform (215) gemäß der Achse der Prothese (1) versetzt sind, so dass die Ausbildung von vertikalen Rillen im Knochen verhindert wird.

10. Intramedulläre Prothese gemäß einem der Ansprüche 5 und 6, **dadurch gekennzeichnet, dass** die Verzahnungen eine Spanbildungs-Verzahnung bilden, die den Unebenheiten eine Schneidewirkung im Knochen verleiht.
